# EUROPEAN PATENT SPECIFICATION

(11) **EP 0 251 431 B1**
(45) Date of publication and mention of the grant of the patent: **28.07.1993**
(21) Application number: 87302734.6
(22) Date of filing: 30.03.1987
(51) Int. Cl.: C08G 59/06, C07D 303/16

(54) **Epoxy resin**
Epoxyharz
Résine époxy

(30) Priority: 24.06.1986 JP 147808/86; 24.12.1986 JP 306561/86
(43) Date of publication of application: 07.01.1988
(73) Proprietor: MITSUI PETROCHEMICAL INDUSTRIES, LTD., Tokyo 100 (JP)
(72) Inventor: Takata, Toshimasa, Ichihara-Shi, Chiba-ken (JP); Mizuno, Kenichi, Iwakuni-shi Yamaguchi-ken (JP)
(74) Representative: Goldin, Douglas Michael

(56) References cited:
- EP-A- 103 282
- DE-B- 2 521 813
- GB-A- 871 301
- GB-A- 991 997
- GB-A- 1 019 130
- US-A- 3 277 048
- US-A- 4 552 814

## Description

The present invention relates to an epoxy resin composition. More particularly, the present invention relates to an epoxy resin composition excellent in heat resistance and flame retardancy, which is obtained by reacting a specific trifunctional epoxy compound with a halogenated bisphenol. Furthermore, the present invention relates to a novel epoxy resin excellent in heat resistance and mechanical properties.

Compositions formed by incorporating a curing agent such as an aromatic polyamine, an aliphatic polyamine, a polyamide-amine, an amine adduct, dicyandiamide, an acid anhydride or a phenol-novolak resin into an epoxy resin have been used as adhesives, paints, molding materials and casting materials. Moreover, it is known that a varnish is formed by dissolving such a composition in a solvent, a reinforcing substrate is impregnated or coated with this varnish and the impregnated or laminated reinforcing substrate is used for molding a laminated plate.

Recently, in the electrical and electronic fields, with required reduction of the size and required increase of precision, improvement of heat resistance is eagerly desired in adhesives, insulating paints, sealants and laminated plates for electronic parts for enhancing the reliability at high-temperature applications More specifically, in an adhesive, paint, sealant or laminated plate prepared by using a commercially available bisphenol A type epoxy resin is generally low in the heat distortion temperature or electrically insulating property, and therefore, the reliability is poor.

Furthermore, a high flame retardancy is required for a material to be used in the electrical or electronic field. As the flame-retardant epoxy resin to be used as a laminated plate (printed circuit substrate comprising a laminate of a glass cloth and an epoxy resin), there is known, for example, a linear epoxy resin obtained by reacting a bisphenol A type epoxy resin such as a liquid bisphenol A type epoxy resin having an epoxy equivalent of about 190 with tetrabromobisphenol A. When this linear epoxy resin is cured with, for example, dicyandiamide which is a curing agent used for formation of a laminated plate and having a high general-purpose property, the glass transition temperature (Tg) of the cured product (the bromine content is 20 to 22 % by weight) is as low as 120 to 130°C. if a large amount of a polyfunctional epoxy resin such as o-cresol-novolak epoxy resin or a phenol-novolak epoxy resin is added to the above epoxy resin so as to increase the heat resistance of the cured product, the flame retardancy is reduced and the moldability becomes insufficient, and therefore, the amount added of the polyfunctional epoxy resin is restricted.

As is apparent from the foregoing description, in the known epoxy resins, heat resistance and flame retardancy are properties contradictory to each other, and an epoxy resin excellent in both heat resistance and flame retardancy is not known. In the electronic field where high performance is required, in order to improve the reliability of a cured product at high temperatures, development of an epoxy resin excellent in both the heat resistance and flame retardancy is eagerly desired.

A cured product formed from a polyfunctional epoxy resin such as an o-cresol-novolak epoxy resin or a phenol-novolak resin has a high flexural modulus and hence, is hard and brittle, and therefore, the cured product is poor in mechanical properties and cracking is readily caused by a thermal shock. Accordingly, development of an epoxy resin excellent in both heat resistance and mechanical properties is eagerly desired
We found that an epoxy resin composition obtained by reacting a trifunctional epoxy compound having a specific structure with a halogenated bisphenol A in the presence of a catalyst has excellent heat resistance and excellent flame retardancy in combination. It also was found that a trifunctional epoxy resin having a specific structural formula has excellent heat resistance and excellent mechanical properties in combination.

It is a primary object of the present invention to provide a novel epoxy resin composition which can be formed into an insulating paint, sealant or molded product excellent in both heat resistance and flame retardancy by curing.

Another object of the present invention is to provide a novel epoxy resin composition which can be formed into a laminated plate having an improved reliability of the mechanical strength or electrically insulating property at high temperatures by curing.

Still another object of the present invention is to provide a novel epoxy resin which can be formed into a paint, sealant or molded product excellent in heat resistance and mechanical characteristics and having a high resistance to cracking by a thermal shock by curing.

In accordance with the present invention, there is provided a trifunctional epoxy compound of formula (I) 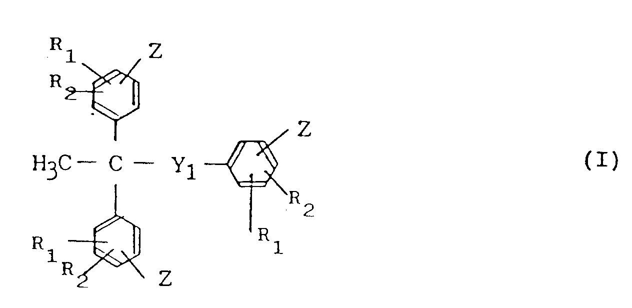
wherein R₁ and R₂ are the same or different and each is hydrogen, halogen, alkyl of 1 to 4 carbon atoms or alkoxy of 1 to 4 carbon atoms (the groups R₁ and R₂ bonded to the respective phenyl groups may be the same or different), Z is a group of formula (II): 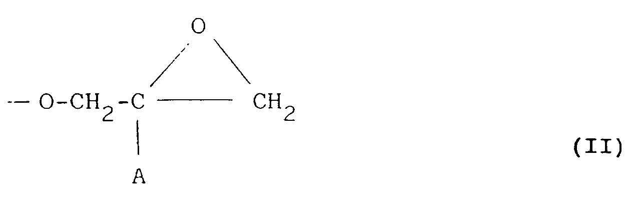
in which A is hydrogen or methyl and Y₁ is a group of formula (III): 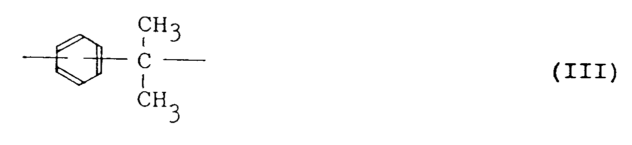
The invention further provides an epoxy resin comprising residues of (A) a trifunctional compound of formula (I) as hereinbefore defined and (B) a halogenated bisphenol.

Since the epoxy resin of the present invention is an epoxy resin obtained by reacting the above-mentioned specific trifunctional epoxy compound with a halogenated bisphenol, as is apparent from comparison of application examples given hereinafter with comparative application examples given hereinafter, a cured product prepared from the epoxy resin of the present invention has a much higher heat resistance than that of a cured product prepared from a known flame-retardant epoxy resin, and since the epoxy resin of the present invention contains a halogen, the cured product shows an excellent flame retardancy.

Accordingly, if the epoxy resin of the present invention is laminated with, for example, a glass cloth and is cured, there can be obtained a laminate which is excellent in both heat resistance and flame retardancy and has an improved reliability at high temperatures and which is very valuable as an electronic part. Moreover, when the novel epoxy resin of the present invention is cured, there can be provided a cured product having a higher flexural strength and a lower flexural modulus than those of a cured product of a conventional epoxy resin and also having a heat distortion temperature higher than 230°C.

Therefore, according to the present invention, there is provided an epoxy resin which gives a cured product having a high reliability even at high temperatures when the epoxy resin is used as an adhesive, a paint, a sealant, a molding material, a varnish or a material for formation of a laminated plate.

Preferred epoxy resins of the invention are those which are soluble in methyl ethyl ketone.

Although the epoxy resin is derived from a trifunctional epoxy compound, the epoxy resin has a substantially linear, gel-free structure such that the epoxy resin is completely soluble in an organic solvent such as methylethylketone, and the epoxy resin of the present invention is advantageous in that the handling and processing properties are very good then the epoxy resin is used in various fields.

Fig. 1 shows an infrared absorption spectrum of an epoxy resin obtained in Example 1, which is a novel compound included within the scope of the trifunctional epoxy compound of the present invention.

Fig. 2 shows an infrared absorption spectrum of an epoxy resin obtained in Example 2, which is a novel compound included within the scope of the trifunctional epoxy compound of the present invention.

The trifunctional epoxy compound used for obtaining the epoxy resin of the present invention is represented by the above-mentioned general formula (I), and it is preferred that this trifunctional epoxy compound be a compound represented by the general formula (I) wherein Z is glycidoxy, R₁ and R₂ are the same or different and each is hydrogen or alkyl of 1 to 4 atoms.

As preferred examples of the trifunctional epoxy compound represented by the general formula (I), there can be mentioned
1-[α-methyl-α-(4'-glycidoxyphenyl)ethyl]-4-[α',α'-bis(4''-glycidoxyphenyl)-ethyl]benzene, 1-[α-methyl-α-(2'-methyl-4'-gylcidoxy-5'tert-butylphenyl)ethyl]-4-[α',α'-bis(2''-methyl-4''-glycidoxy-5''-tert-butylphenyl)ethyl] benzene, 1-[α-methyl-α-(3',5'-dimethyl-4'-glycidoxyphenyl)ethyl]-4-[α',α'-bis(3'',5''-dimethyl-4''-glycidoxyphenyl]ethyl]benzene, 1-[α-methyl-α-(3'-tert-butyl-4'-glycidoxyphenyl)ethyl]-4-[α',α'-bis(3''-tert-butyl-4''-glycidoxyphenyl)ethyl)benzene, 1-[α-methyl-α-(3'-methyl-4'-glycidoxy-5'-tert-butylphenyl)ethyl]-4-[α',α'-bis(3''-methyl-4''-glycidoxy-5''-tert-butylphenyl)ethyl)-benzene and 1-[α-methyl-α-(2',5'-dimethyl-4'-glycidoxyphenyl)ethyl]-4-[α',α'-bis(2'',5''-dimethyl-4''-glycidoxyphenyl)ethyl]benzene.

Preferred compounds of formula (I) are 1-[α-methyl-α-(4'-glycidoxyphenyl)ethyl]-4-[α',α'-bis(4''-glycidoxyphenyl)-ethyl]benzene, and 1-[α-methyl-α-(3',5'dimethyl-4'-glycidoxyphenyl)ethyl]-4-[α',α'-bis(3''-5''-dimethyl-4''-glycidoxyphenyl)ethyl]benzene.

In the general formula (I), it is preferred that the group Z be bonded to the paraposition of the phenyl group and R₁ and R₂ should stand for a hydrogen atom or an alkyl group having 1 to 4 carbon atoms.

The trifunctional epoxy compound represented by the general formula (I) is prepared, for example, by etherifying a tris-phenol compound represented by the following general formula (IV): 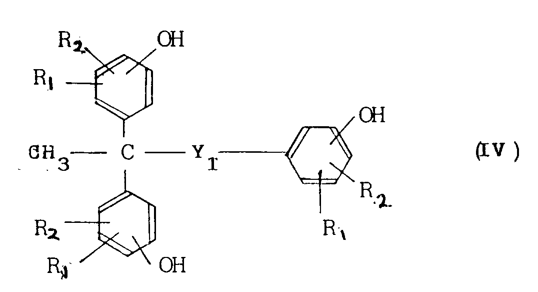
wherein R₁, R₂ and Y, are as defined above, with an epihalohydrin or a β-methylepihalohydrin, preferably epichlorohydrin or β-methylepichlorohydrin, in the presence of an appropriate etherifying catalyst, and dehydrohalogenating the etherification product.

If the tris-phenol compound is reacted with an epihalohydrin or a β-methylepihalohydrin, not only the trifunctional epoxy compound represented by the general formula (I) but also a polyfunctional epoxy compound such as a tetrafunctional epoxy compound represented by the following general formula ( V ): 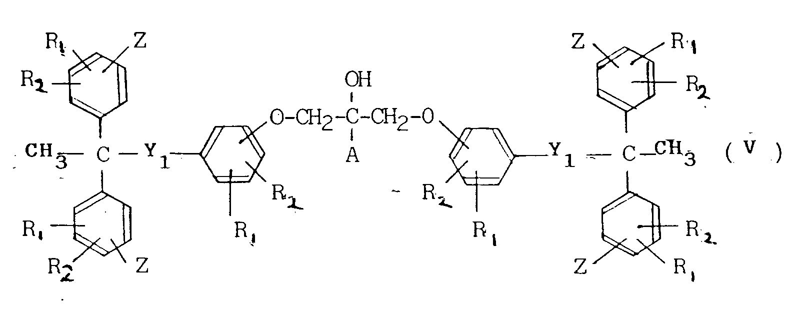
wherein R₁, R₂, , Z. Y₁, and A are as defined above,
is formed. In the present invention, even if a polyfunctional epoxy compound mixture comprising the trifunctional epoxy compound represented by the general formula (I) and other polyfunctional epoxy compound obtained by reacting the tris-phenol-compound represented by the general formula (IV) with an epihalohydrin or a β-methylepihalohydrin is used, a heat-resistant flame-retardant epoxy resin can be obtained by reacting this mixture with a halogenated bisphenol. Accordingly, the following description made with reference to the trifunctional epoxy compound which is the main component of the composition of the present invention will also hold good with respect to the above-mentioned polyfunctional epoxy compound mixture.

The reaction of the tris-phenol compound with the epihalohydrin or β-methylepihalohydrin can be accomplished according to various known processes. For example, there can be adopted a process in which an alkali compound, for example, an alkali metal hydroxide such as sodium hydroxide, potassium hydroxide or lithium hydroxide, preferably sodium hydroxide, is used in an amount of at least 1 mole, preferably 1.02 to 1.05 mole, per equivalent of the phenolic hydroxyl group of the tris-phenol, etherification and dehydrohalogenation are simultaneously carried out in the presence of water at a temperature of about 60 to about 90°C, the unreacted halohydrin, water and the formed salt are removed from the reaction mixture after the reaction and the trifunctional epoxy compound as the reaction product is dried and recovered.

However, a process in which etherification and dehydrohalogenation are carried out in sequence is preferred because an epoxy compound having a stable quality can be obtained.

The etherification is carried out in the presence of about 0.005 to 5 mole %, based on 1 equivalent of the phenolic hydroxyl group of the tris-phenol, of an etherifying catalyst, for example, a tertiary amine such as trimethylamine or triethylamine, a tertiary phosphine such as triphenylphosphine or tributylphosphine, a quaternary ammonium salt such as tetramethyl ammonium chloride, tetramethyl ammonium bromide, tetraethyl ammonium chloride, tetraethyl ammonium bromide or choline chloride, a quaternary phosphonium salt such as tetramethyl phosphonium bromide, tetramethyl phosphonium iodide or triphenylpropyl phosphonium bromide or a tertiary sulfonium salt such as benzyldibutyl sulfonium chloride or benzyldimethyl sulfonium chloride, preferably a quaternary ammonium salt.

At the etherification step, the reaction is carried out to such an extent that at least 50 %, preferably at least 80 %, of the hydroxyl group of the tris-phenol be etherified. The reaction is generally conducted at a temperature of 60 to 110°C for 1 to 12 hours. The presence of water is not preferred. If water is present, the amount of water should be controlled below 3.0 % by weight.

At the subsequent dehydrohalogenation step, the reaction product obtained at the etherification step is subjected to the reaction as it is, that is, in the state where the reaction product contains the unreacted epihalohydrin. As the catalyst for the reaction, there is used the same alkali compound as used in the above-mentioned first process, for example, an alkali metal hydroxide, preferably sodium hydroxide, in an amount of at least 0.5 mole, preferably at least 0.8 mole, per equivalent of the phenolic hydroxyl group of the tris-phenol. However, in order to avoid gelation or the like, the amount of the catalyst should be smaller than 1 mole per equivalent of the phenolic hydroxyl group of the tris-phenol.

It is preferred that the epihalohydrin or β-methylepihalohydrin be used in an amount of 3 to 30 moles per mole of the tris-phenol compound. From the industrial viewpoint, epichlorohydrin or β - methylepichlorohydrin is preferred as the epihalohydrin or β-methylepihalohydrin.

After completion of the reaction, removal of the unreacted epihalohydrin by distillation under reduced pressure, removal of the salt formed as the by-product by water washing or the like and, if necessary, neutralization with a weak acid such as phosphoric acid or sodium dihydrogenphosphate are carried out, and then, drying is carried out to obtain the intended epoxy compound.

The trifunctional compound of the present invention is semi-solid or a solid having a softening point lower than 130°C, and the epoxy equivalent is from 154 to 380.

In view of the effect of improving the flame retardancy and the easy industrial availability, a brominated bisphenol is preferred as the halogenated bisphenol to be reacted with the trifunctional epoxy compound in the present invention. For example, tetrabromobisphenol A, tetrabromobisphenol B, tetrabromobisphenol F and 1,1-bis(3,5-dibromo-4-hydroxyphenyl)ethane are especially preferred.

In the present invention, the reaction of the trifunctional epoxy compound with the halogenated bisphenol is carried out in the presence of a catalyst in the absence of a solvent or, if necessary, in a solvent, for example, an aromatic solvent such as toluene or xylene or a ketone solvent such as methylisobutyl ketone.

Any known catalysts customarily used for the polyaddition reaction between the epoxy group and the phenolic hydroxyl group can be used as the catalyst in the present invention. For example, there can be mentioned basic catalysts such as sodium hydroxide and sodium carbonate, quaternary ammonium salt catalysts such as tetra-alkyl ammonium halides and aralkyl-trialkyl ammonium halides and phosphorus type catalysts such as triphenylphosphine and ethyltriphenyl phosphonium halides. It is preferred that the catalyst be used in an amount of 10 to 400 ppm based on the used trifunctional epoxy compound.

The above reaction can be carried out at a temperature of 120 to 200°C under atmospheric pressure for 3 to 20 hours with stirring in the molten state or solution state.

In the above reaction, a difunctional epoxy compound such as a bisphenol A type epoxy resin, a bisphenol F type epoxy resin or 1,1-bis(glycidoxyphenyl)ethane may be present in the reaction system. In the epoxy resin composition of the present invention obtained by reacting the trifunctional epoxy compound with the halogenated bisphenol, as the halogen content is high, the softening point tends to be too high. However, if the above-mentioned difunctional epoxy compound is used in combination with the trifunctional epoxy compound, the softening point of the obtained epoxy resin can be reduced without degradation of the flame retardancy. Accordingly, the amount of the difunctional epoxy compound used can be appropriately adjusted according to the halogen content of the epoxy resin composition of the present invention and the desired halogen content. However, in order to improve the glass transition temperature of the cured product obtained from the epoxy resin composition of the present invention over that of a cured product obtained from a known epoxy resin, it is preferred that the weight ratio of the trifunctional epoxy compound to the difunctional epoxy compound be in the range of from 50/50 to 90/10, especially from 60/40 to 80/20.

The reaction ratio of the epoxy compound to the halogenated bisphenol is appropriately selected according to the desired halogen content in the epoxy resin composition obtained by the reaction. However, it is generally preferred that the reaction ratio be selected so that the halogen content in the epoxy resin composition of the present invention obtained by the reaction is 5 to 30 % by weight, especially 10 to 25 % by weight, particularly especially 15 to 20 % by weight. If the halogen content in the epoxy resin composition is too low and below the above-mentioned range, no sufficient flame retardancy can be attained.

The invention therefore provides a process for producing the foregoing epoxy resins comprising reacting a trifunctional epoxy compound of formula (I) with a halogenated bisphenol in the presence of a catalyst, preferably involving reacting a bisphenol with an epihalohydrin and reacting the condensation product with the trifunctional epoxy compound and halogenated bisphenol.

In order to attain the objects of the present invention, it is preferred that the epoxy equivalent of the final epoxy resin be 300 to 2000, especially 300 to 1000.

The epoxy resin composition of the present invention comprises a polymer represented by the following general formula (VI)), though the general formula is not particularly critical: 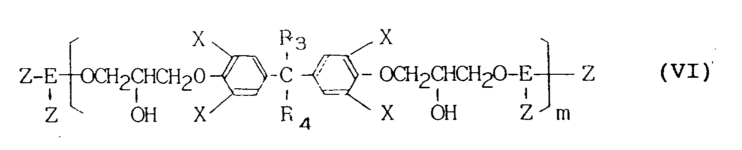
wherein X is glycidoxy, R₃ and R₄ are the same or different and each is hydrogen or alkyl of 1 to 4 carbon atoms, X is halogen, E is a group of formula (VII) 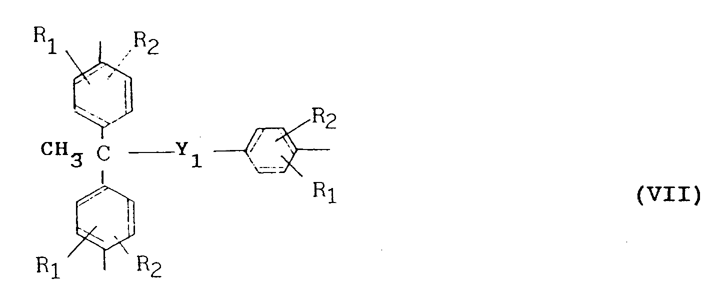
in which R₁ and R₂ are the same or different and each is hydrogen or alkyl of 1 to 4 carbon atoms, Y₁ is a group of formula (III) as hereinbefore defined and m is an integer of at least 1.

Moreover, the epoxy resin composition derived from the combination of the trifunctional epoxy compound and the difunctional epoxy compound comprises a copolymer represented by the following general formula (VIII). 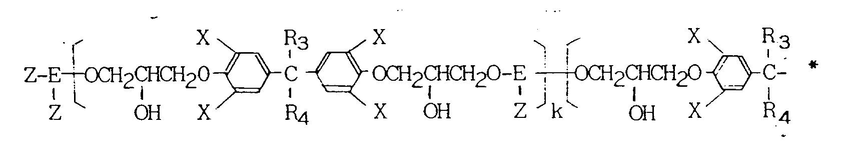 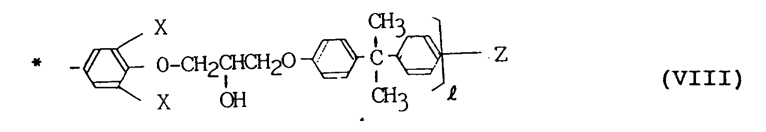
wherein Z is glycidoxy, R₃ and R₄ are the same or different and each is hydrogen or alkyl of 1 to 4 carbon atoms, X is halogen, E is a group of formula (VII): 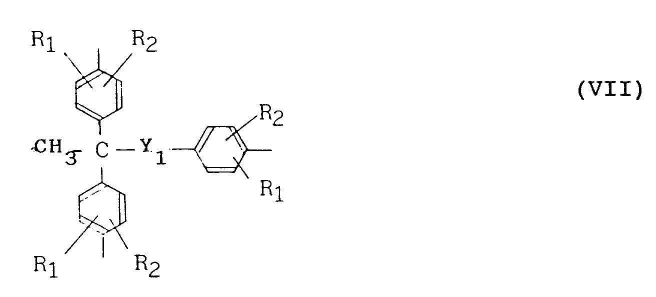
in which R₁ and R₂ are the same or different and each is hydrogen or alkyl of 1 to 4 carbon atoms, Y₁ is a group of formula (III) as hereinbefore defined, k is O or an integer of at least 1, l is an integer of at least 1, and k and l are selected so that the halogen-containing epoxy resin has an epoxy equivalent of 300 to 2000 and a
halogen content of 5 to 30 % by weight.

The epoxy resin composition of the present invention may further comprise a known epoxy resin such as a phenol-novolak type epoxy resin or an o-cresol-novolak type epoxy resin, so far as the attainment of the objects of the present invention is not hindered.

In addition to the foregoing components, the epoxy resin composition of the present invention may comprise an unreactive diluent such as a phthalic acid ester or an ether or ester of a glycol, a reactive diluent such as a long-chain alkylene oxide, butylglycidyl ether, phenylglycidyl ether or p-butylphenyl-glycidyl ether, a filler such as calcium carbonate, clay, asbestos, silica, mica, quartz powder, aluminum powder, graphite, titanium oxide, alumina, iron oxide, glass powder or glass fiber, and a colorant such as carbon black, Toluidine Red, Hansa Yellow, Phthalocyanine Blue or Phthalocyanine Green.

When the epoxy resin composition of the present invention is actually used, a known curing agent for an epoxy resin, for example, an aliphatic amine, an aromatic amine, an amine adduct, dicyandiamide, a phenol-novolak resin, an o-cresol-novolak resin or an acid anhydride is incorporated into the epoxy resin composition, and the composition is used for the production of an electrically insulating paint, a molding material, a sealant, a laminated plate and the like.

The amount used of the curing agent is changed according to the kind of the curing agent. For example, in case of a polyamine, the amount used of the polyamine as the curing agent is determined based on the ratio between the epoxy equivalent and the active hydrogen equivalent. A curing promotor can be used according to need.

When the epoxy resin composition is used for a paint, a general-purpose colorant (pigment), a filler, a solvent, a defoamer and the like are incorporated into the epoxy resin composition, and various fillers can be incorporated for the production of sealants. When the epoxy resin composition is used for the production of a laminated plate, a varnish is generally formed by dissolving the epoxy resin composition in an aromatic hydrocarbon such as toluene or xylene or a ketone type solvent such as acetone, methylethylketone or methylisobutylketone. A reinforcing substrate such as a glass cloth, carbon fiber, glass fiber, paper, asbestos, polyester fiber or aromatic polyamide fiber (such as a product marketed under the tradename of "Kevler") is impregnated with the so-formed varnish to form a prepreg, and the prepregs are heat-pressed to obtain a laminated plate.

The trifunctional epoxy compound represented by the general formula (I) may be mixed with other epoxy resin, a reactive diluent, a filler, a colorant and the like according to need, so far as the characteristics are not degraded, as described hereinbefore with respect to the heat-resistant flame-retardant epoxy resin composition, and the resulting composition is mixed with a curing agent and, if necessary, a curing promotor, and is used for the production of an electrically insulating paint, a sealant, a molding material, an adhesive, a material for formation of a laminated plate and the like.

The present invention will now be described in detail with reference to the following examples that by no means limit the scope of the invention.

### Example 1

A 1-liter four-neck glass flask equipped with a stirring rod and a reflux device was charged with 462.5 g of epichlorohydrin, 141.3 g of 1-[α-methyl-α-(4'-hydroxyphenyl)ethyl]-4-[α',α'-bis(4''-hydroxyphenyl)ethyl] benzene and 2.73 g of tetramethyl ammonium chloride, and reaction was carried out at 70°C for 3 hours with stirring.

While this temperature was being maintained, 79 g of a 48 % aqueous solution of sodium hydroxide (the molar ratio to the tris-phenol was 2.85) was added dropwise to the reaction mixture over a period of 2 hours. The pressure in the system was reduced to 150 to 250 mmHg and water formed by the reaction was removed from the system, and epichlorohydrin azeotropically distilled was returned into the system. Even after the dropwise addition, water was removed from the system until formation of water was not observed. Subsequently, unreacted epichlorohydrin was removed from the reaction mixture by distillation. To the residue were added 230 g of methylisobutylketone and 230 g of water, and the mixture was stirred to transfer Formed sodium chloride into the aqueous phase. Then, the mixture was allowed to stand still and the separated aqueous phase was removed.

Then, 20 g of a 24 % aqueous solution of sodium hydroxide was added to the oil phase and the mixture was stirred at 90°C for 2 hours to effect the second dehydrohalogenation. Then, the oil phase was separated from the aqueous phase and neutralized with 76 g of a 30 % aqueous solution of sodium dihydrogenphosphate, and removal of water by azeotropic distillation and removal of the salt by Filtration using 4G glass filter were carried out.

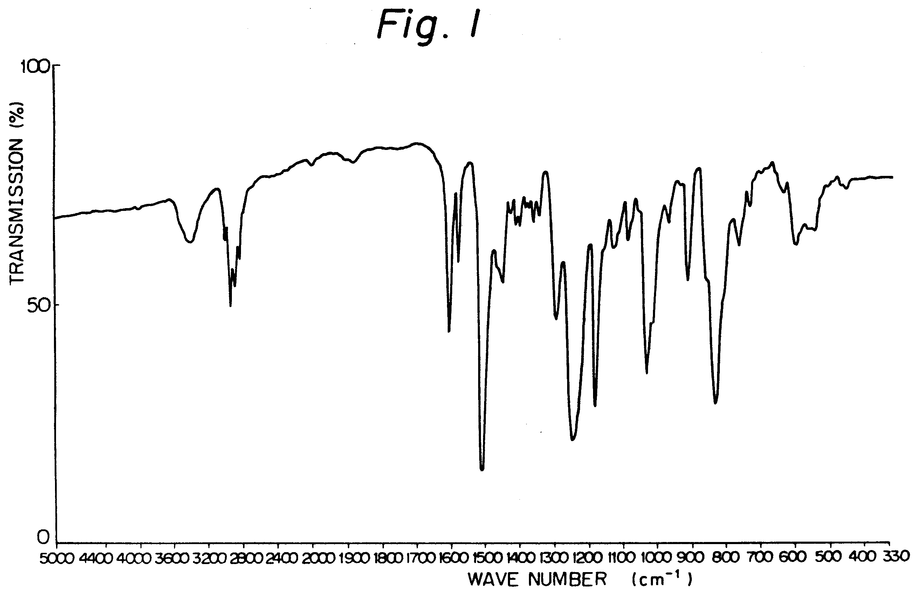

Methylisobutylketone was completely removed from the oil phase under a reduced pressure of 5 mmHg at 150°C to obtain 180 g of 1-[α-methyl-α-(4'-glycidoxyphenyl)ethyl]-4-[α',α'-bis(4''-glycidoxyphenyl)ethyl]benzene having an epoxy equivalent of 219 and a softening point of 63°C. The infrared absorption spectrum of the obtained resin is shown in Fig. 1.

The results of ¹H-NMR (proton nuclear magnetic resonance; CDCℓ₃ solution) are as follows:
- δ =: 1.62, 6H, s; 3.17-3.44, 3H, m; 2.07, 3H, s; 3.77-4.24, 6H, m, 2.64-2.94, 6H, m; 6.69-7.20, 16H, m

### Example 2

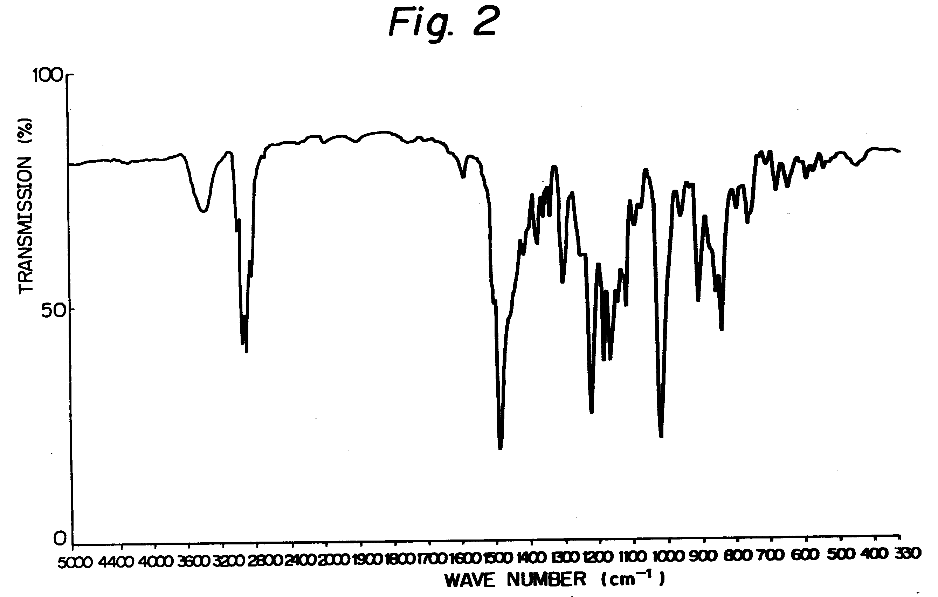

Procedures of Example 1 were repeated in the same manner except that 155.0 g of 1-[α-thyl-α-(3',5'-dimethyl-4'hydroxyphenyl)ethyl]4-[α',α'-bis(3'',5''-dimethyl-4''-hydroxyphenyl)ethyl]benzene was used as the tris-phenol, whereby 196 g of 1-[α-methyl-α-(3',5'-dimethyl-4'-glycidoxyphenyl)ethyl]-4-[α',α'-bis(3'',5''-dimethyl-4''-glycidoxyphenyl)ethyl]benzene having an epoxy equivalent of 240 and a softening point of 87°C was obtained. The infrared absorption spectrum of the obtained epoxy resin is shown in Fig. 2.

The results of ¹H-NMR (proton nuclear magnetic resonance; CDCℓ₃ solution) are as follows:
- δ =: 1.62, 6H, s, 3.18-3.44, 3H, m, 2.06, 3H, s, 3.60-4.15, 6H, m, 2.17, 12H, s, 6.65, 4H, s, 2.21, 6H, s, 6.82, 2H, s, 2.61-2.95, 6H, m, 6.95-7.03, 4H, d

### Application Example 1

A cured product was obtained by heating and mixing 100 parts (parts by weight; all of "parts" given hereinafter are by weight) of the epoxy resin obtained in Example 1 with 78 parts of Methyl Nadic anhydride (Kayahard MCD supplied by Nippon Kayaku) and 1 part of 2-ethyl-4-methylimidazole (2E4MZ supplied by Shikoku Kasei) at 100°C for 3 hours and at 230°C for 2 hours. The obtained cured product was tested with respect to the heat distortion temperature (ASTM D-648), the flexural strength (JIS K-6911) and the flexural modulus (JIS K-6911).

### Application Example 2

In the same manner as described in Application Example 1, a cured product was prepared from 50 parts of the epoxy resin obtained in Example 1, 50 parts of a bisphenol A type epoxy resin (EPOMIK R-140 supplied Mitsui Sekiyu Kagaku), 84 parts of Methyl Nadic anhydride and 1 part of 2-ethyl-4-methylimidazole and the obtained cured product was tested.

### Application Example 3

In the same manner as described in Application Example 1, a cured product was prepared from 100 parts of the epoxy resin obtained in Example 2, 71 parts of Methyl Nadic anhydride and 1 part of 2-ethyl-4-methylimidazole and the cured product was tested.

### Comparative Application Example 1

In the same manner as described in Application Example 1, a cured product was prepared from 100 parts of a bisphenol A type epoxy resin (EPOMIK R-140), 90 parts of Methyl Nadic anhydride and 1 part of 2-ethyl-4-methylimidazole and the cured product was tested.

### Comparative Application Example 2

In the same manner as described in Application Example 1, a cured product was prepared from 100 parts of an o-cresolnovolak type epoxy resin (EOCN 102 supplied by Nippon Kayaku), 81 parts of Methyl Nadic anhydride and 1 part of 2-methyl-4-methylimidazole and the cured product was tested.

The results obtained in the application examples and comparative application examples are shown in Table 1.

**Table 1**

| | Heat Distortion Temperature (°C) | Flexural Strength (Kg/mm²) | Flexural Modulus (Kg/mm²) |
|---|---|---|---|
| Application Example 1 | 236 | 11.7 | 283 |
| Application Example 2 | 198 | 12.6 | 287 |
| Application Example 3 | 239 | 10.5 | 296 |
| Comparative Application Example 1 | 169 | 13.0 | 299 |
| Comparative Application Example 2 | 235 | 7.8 | 322 |

### Comparative Example 1

A 1-liter separable flask was charged with 400 g of 1,1,3-tris(2-methyl-4-glycidoxy-5-tert-butylphenyl)butane (having an epoxy equivalent of 248), 176.4 g of tetrabromobisphenol A and 60 g of xylene, and 1.6 mℓ of an aqueous solution containing 1 %, by weight of tetramethyl ammonium chloride was further added. When the mixture was heated with stirring in a nitrogen gas atmosphere, the mixture became completely homogeneous at a temperature of up to 100°C. Then, the pressure was reduced and xylene and water were removed while elevating the temperature to 140°C. The pressure was returned to atmospheric pressure and the reaction mixture was further heated at 150°C for 6 hours in a nitrogen gas atmosphere.

As the result, 576.4 g of an epoxy resin composition having an epoxy equivalent of 605 and a bromine content of about 18 %, by weight was obtained. This epoxy resin composition was soluble in methylethylketone.

### Comparative Example 2

Procedures of Example 3 were repeated in the same manner except, that a mixture of 280 g of 1,1,3-tris(2-methyl-4-glycidoxy-5-tert-butylphenyl)butane (having an epoxy equivalent of 271) and 120 g of a bisphenol A type epoxy resin (having an epoxy equivalent of 189; EPOMIK R-140 supplied by Mitsui Sekiyu Kagaku) was used instead of 400 g of 1,1,3-tris(2-methyl-4-glycidoxy-5-tert-butylphenyl)butane, whereby 576.4 g of an epoxy resin composition having an epoxy equivalent of 573 and a bromine content of about 18 % by weight was obtained. This epoxy resin composition was soluble in methylethylketone.

### Example 3

Procedures of Comparative Example 1 were repeated in the same manner except that 400 g of 1-[α-methyl-α-(4'-glycidoxyphenyl)ethyl]-4-[α',α'-bis(4''-glycidoxyphenyl)ethyl)benzene (having an epoxy equivalent of 209) was used instead of 400 g of 1,1-3-tris(2-methyl-4-glycidoxy-5-tert-butylphenyl)butane, whereby 576.4 g of an epoxy resin composition having an epoxy equivalent of 486 and a bromine content of about 18 % by weight was obtained. This epoxy resin composition was soluble in methylethylketone.

### Example 4

Procedures of Comparative Example 1 were repeated in the same manner except that a mixture of 280 g of 1-[α-methyl-α-(4'-glycidoxyphenyl)ethyl)-4-[α',α'-bis(4''- glycidoxyphenyl)ethyl)benzene (having an epoxy equivalent of 217) and 120 g of a bisphenol A type epoxy resin (having an epoxy equivalent of 189; EPOMIK R-140 supplied by Mitsui Sekiyu Kagaku) was used instead of 400 g of 1,1,3-tris(2-methyl-4-glycidoxy-5-tert-butylphenyl)butane, whereby 576.4 g of an epoxy resin composition having an epoxy equivalent of 453 and a bromine content of about 18 % by weight was obtained. This epoxy resin composition was soluble in methylethylketone.

### Example 5

Procedures of Comparative Example 1 were repeated in the same manner except that 400 g of 1-[α-methyl-α-(3',5'-dimethyl-4'-glycidoxyphenyl)ethyl]-4-[α',α'-bis(3'',5''-dimethyl-4''-glycidoxyphenyl)-ethyl]benzene (having an epoxy equivalent of 240) was used instead of 400 g of 1,1,3-tris(2-methyl-4-glycidoxy-5-tert-butylphenyl)butane, whereby 576.4 g of an epoxy resin composition having an epoxy equivalent of 451 and a bromine content of about 18 % by weight was obtained. This epoxy resin composition was soluble in methylethylketone.

### Comparative Example 3

Procedures of Comparative Example 1 were repeated in the same manner except that 400 g of tris(4-glycidoxyphenyl)methane (having a epoxy equivalent of 162) was used instead of 400 g of 1,1,3-tris(2-methyl-4-glycidoxy-5-tert-butylphenyl)butane, whereby 576.4 g of an epoxy resin composition having an epoxy equivalent of 330 and a bromine content of about 18 % by weight was obtained. This epoxy resin composition was soluble in methylethylketone.

### Comparative Example 4

Procedures of Comparative Example 1 were repeated in the same manner except that 400 g of 1,1,3-tris(4-glycidoxyphenyl)propane (having an epoxy equivalent of 186) was used instead of 400 g of 1,1,3-tris(2-methyl-4-glycidoxy-5-tert-butylphenyl)butane, whereby 576.4 g of an epoxy resin composition having an epoxy equivalent of 394 and a bromine content of about 18 % by weight was obtained. This epoxy resin composition was soluble in methylethylketone.

### Comparative Example 5

Procedures of Comparative Example 1 were repeated in the same manner except that 400 g of 1,1,3-tris(3,5-dimethyl-4-glycidoxyphenyl)propane (having an epoxy equivalent of 206) was used instead of 400 g of 1,1,3-tris(2-methyl-4-glycidoxy-5-tert-butylphenyl)butane, whereby 576.4 g of an epoxy resin composition having an epoxy equivalent of 446 and a bromine content of about 18 % by weight was obtained. This epoxy resin composition was soluble in methylethylketone.

### Comparative Example 6

Procedures of Comparative Example 1 were repeated in the same manner except that 400 g of an o-cresol-novolak type epoxy resin (having an epoxy equivalent of 212; EOCN 102 supplied by Nippon Kayaku) was used instead of 400 g of 1,1,3-tris(2-methyl-4-glycidoxy-5-tert-butylphenyl)butane. When the reaction was conducted for several hours, the reaction product was gelled, and the obtained reaction product was insoluble in methylethylketone.

### Comparative Application Example 3

The epoxy resin composition obtained in Comparative Example 1 was dissolved in methylethylketone to form a solution having an epoxy resin concentration of 75 % by weight. This epoxy resin solution (100 parts by weight as the solid) was mixed with a solution comprising 15 parts by weight of methyl cellosolve, 15 parts by weight of dimethylformamide, 3.1 parts by weight of dicyandiamide and 0.2 part by weight of 2-ethyl-4-methyl-imidazole (2E4MZ supplied by Shikoku Kasei) to form a vanish-like epoxy resin composition.

A glass cloth (WE-18K-BZ2 supplied by Nitto Boseki) was impregnated with this composition and was heated at 150°C for 6 minutes to obtain a prepreg of the B stage having a resin impregnation ratio of about 45 % by weight. These prepregs were piled in 9 plies and a glass cloth laminated plate was formed under molding conditions of 180°C, 10 Kgf/cm² and 90 minutes.

The glass transition temperature (Tg) of the cured resin of the obtained laminated plate was 185°C as measured by a differential scanning calorimeter (DSC), and the flame retardancy was 94V-O as determined by the UL method.

### Comparative Application Example 4

A glass cloth laminated plate was prepared by repeating procedures of Comparative Application Example 3 in the same manner except that the epoxy resin composition obtained in Comparative Example 2 was used instead of the epoxy resin composition obtained in Comparative Example 1 and the amount used of dicyandiamide was changed to 3.3 parts by weight.

Tg of the cured resin of the laminated plate was 170°C and the flame retardancy was 94V-O as determined by the UL method.

### Application Example 4

A glass cloth laminated plate was prepared by repeating procedures of Comparative Application Example 3 in the same manner except that the epoxy resin composition obtained in Example 3 was used instead of the epoxy resin composition obtained in Comparative Example 1 and the amount used of dicyandiamide was changed to 3.9 parts by weight.

Tg of the cured resin of the laminated plate was 188°C.

### Application Example 5

A glass cloth laminated plate was prepared by repeating procedures of Comparative Application Example 3 in the same manner except that the epoxy resin composition obtained in Example 4 was used instead of the epoxy resin composition obtained in Comparative Example 1 and the amount used of dicyandiamide was changed to 4.2 parts by weight.

Tg of the cured resin of the laminated plate was 175°C, and the flame retardance was 94V-O as determined by the UL method.

### Comparative Application Example 5

A glass cloth laminated plate was prepared by repeating procedures of Comparative Application Example 3 in the same manner except the 100 parts by weight of a brominated bisphenol A type epoxy resin (having an epoxy equivalent of 408 and a bromine content of 18 % by weight) was used instead of the epoxy resin composition obtained in Comparative Example 1 and the amount used of dicyandiamide was changed 4.7 parts by weight.

Tg of the cured resin of the laminated plate was 127°C.

### Application Example 6

A glass cloth laminated plate was prepared by repeating procedures of Comparative Application Example 1 in the same manner except that the epoxy resin composition obtained in Example 5 was used instead of the epoxy resin composition obtained in Comparative Example 1 and the amount used of dicyandiamide was changed to 4.2 parts by weight.

Tg of the cured resin of the laminated plate was 190°C and the flame retardancy was 94V-0 as determined by the UL method.

### Comparative Application Example 6

A glass cloth laminated plate was prepared by repeating procedures of Comparative Application Example 3 in the same manner except that the epoxy resin composition obtained in Comparative Example 3 was used instead of the epoxy resin composition obtained in Comparative Example 1 and the amount used of dicyandiamide was changed to 5.8 parts by weight.

Tg of the cured resin of the laminated plate was 181°C and the flame retardancy was 94V-0 as determined by the UL method.

### Comparative Application Example 7

A glass cloth laminated plate was prepared by repeating procedures of Comparative Application Example 3 in the same manner except that the epoxy resin composition obtained in Comparative Example 4 was used instead of the epoxy resin composition obtained in Comparative Example 1 and the amount used of dicyandiamide was changed to 4.8 parts by weight.

Tg of the cured resin of the laminated plate was 181°C and the flame retardancy was 94V-0 as determined by the UL method.

### Comparative Application Example 8

A glass cloth laminated plate was prepared by repeating procedures of Comparative Application Example 3 in the same manner except that the epoxy resin composition obtained in Comparative Example 5 was used instead of the epoxy resin composition obtained in Comparative Example 1 and the amount used of dicyandiamide was changed to 4.4 parts by weight.

Tg of the cured resin of the laminated plate was 183°C and the flame retardancy was 94V-O as determined by the UL method.

### Comparative Application Example 9

A glass cloth laminated plate was prepared by repeating procedures of Application Example 4 in the same manner except that a mixture (having a bromine content of about 18 % by weight) comprising 86 parts by weight of a brominated bisphenol A type epoxy resin (having an epoxy equivalent of 479 and a bromine content of 21 % by weight) and 14 parts by weight of an o-cresol-novolak type epoxy resin (having an epoxy equivalent of 210; EOCN 103S supplied by Nippon Kayaku) was used instead of the epoxy resin composition obtained in Comparative Example 1.

Tg of the cured resin of the laminated plate was 137°C.

## Claims

1. A trifunctional epoxy compound of formula (I) 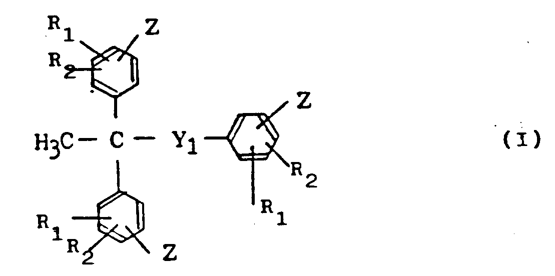 wherein R₁ and R₂ are the same or different and each is hydrogen halogen, alkyl of 1 to 4 carbon atoms or alkoxy of 1 to 4 carbon atoms, the groups R₁ and R₂ bonded to the respective phenyl groups may be the same or different, Z is a group of formula (II): 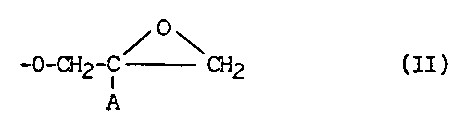 in which A is hydrogen or methyl and Y₁ is a group of formula (III)

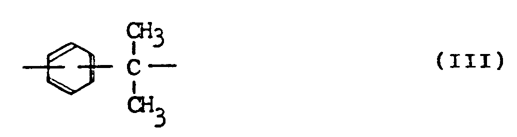

2. 1-[α-Methyl-α-(4'-glycidoxyphenyl)ethyl]-4-[α',α'-bis(4''-glycidoxyphenyl)ethyl)benzene.

3. 1-[α-Methyl-α-(3',5'-dimethyl-4'-glycidoxyphenyl)ethyl]-4-[α',α'-bis(3'',5''-dimethyl-4''- glycidoxyphenyl)ethyl]bezene.

4. An epoxy resin comprising residues of (A) a trifunctional epoxy Compound of formula (I): 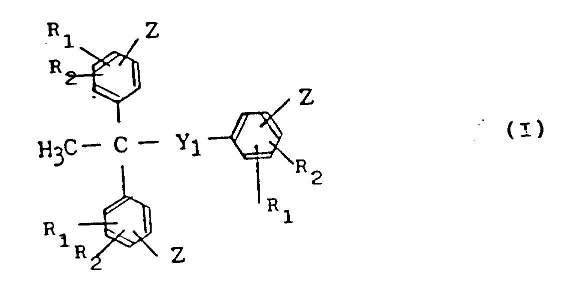 wherein R₁ and R₂ are the same or different and each is hydrogen, halogen, alkyl or 1 to 4 carbon atoms or alkoxy of 1 to 4 carbon atoms, the groups R₁ and R₂ bonded to the respective phenyl groups may be the same or different, Z is a group of formula (II): 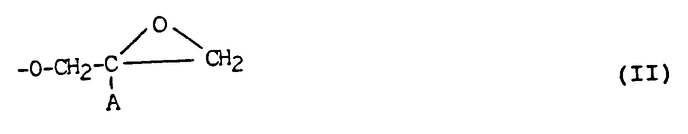 in which A is hydrogen or methyl and Y₁ is a group of formula (III) 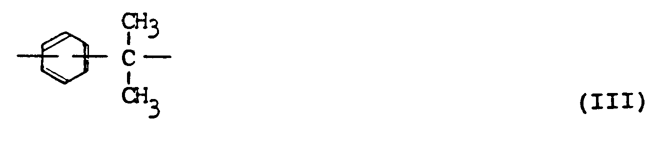 and (B) residues of a halogenated bisphenol.

5. An epoxy resin according to claim 4 comprising residues of the difunctional epoxy condensation product of a bisphenol and an epihalohydrin.

6. A epoxy resin according to claim 4 or claim 5 wherein the trifunctional epoxy compound is a compound of formula (I) wherein Z is glycidoxy, R₁ and R₂ are the same or different and each is hydrogen or alkyl of 1 to 4 carbon atoms and Y₁ is as defined in claim 4.

7. An epoxy resin according to any one of claims 4 to 6 having an epoxy equivalent of 300 or 2000.

8. An epoxy resin according to any one of claims 4 to 7 having a halogen content of 5 to 30% by weight.

9. An epoxy resin according to any one of claims 4 to 8 wherein the halogenated bisphenol is tetrabromobisphenol A.

10. An epoxy resin according to any one of claims 5 to 9 wherein the weight ratio of the residues of the trifunctional epoxy compound to the residues of the difunctional epoxy condensation product is in the range of from 50/50 to 90/10.

11. An epoxy resin according to claim 10 wherein the weight ratio of the residues of the trifunctional epoxy compound to the residues of the condensation product is in the range of from 60/40 to 80/20.

12. An epoxy resin according to any one of claims 4 to 11 which is soluble in methylethylketone.

13. An epoxy resin of formula (VI) 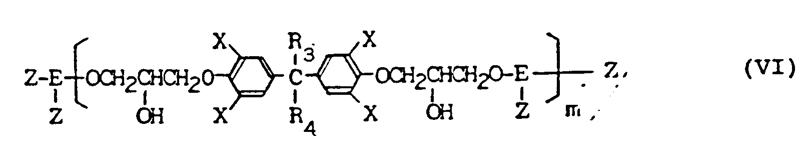 wherein Z is glycidoxy, R₃ and R₄ are the same or different and each is hydrogen or alkyl of 1 to 4 carbon atoms, X is halogen, E is a group of formula (VIII): 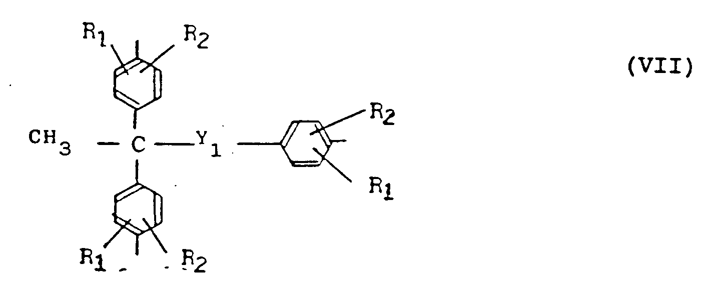 in which R₁ and R₂ are the same or different and each is hydrogen or alkyl of 1 to 4 carbon atoms the groups R₁ and R₂ bonded to the respective phenyl groups may be the same or different, Y₁ is a group of formula (III): 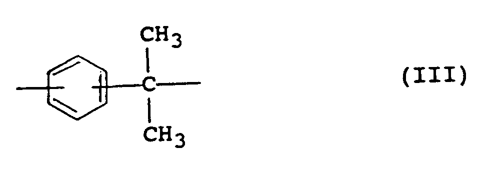 and m is an integer of at least 1, having an epoxy equivalent of 300 to 2000 and a halogen content of 5 to 30% by weight.

14. An epoxy resin comprising residues of formula (VIIIa): 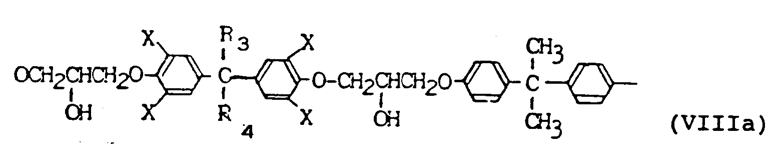 wherein R₃ and R₄ are the same or different and each is hydrogen or alkyl of 1 to 4 carbon atoms, X is halogen and residues of formula (VIIIb) 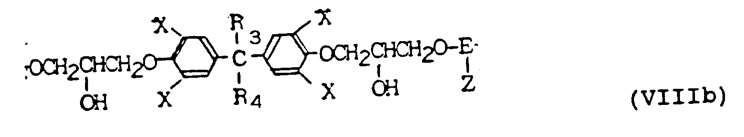 wherein R₃ and R₄ are as defined above, E is a group of formula (VII) 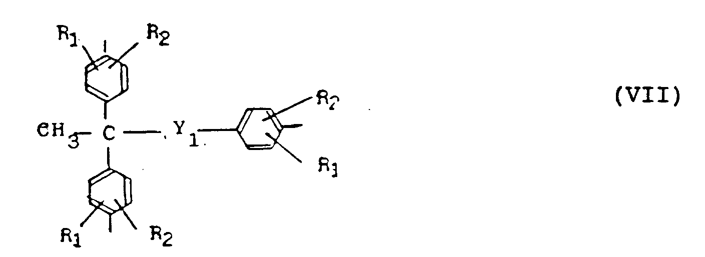 in which R₁ and R₂ are the same or different and each is hydrogen or alkyl of 1 to 4 carbon atoms, the groups R₁ and R₂ bonded to the respective phenyl groups may be the same or different, Y₁ is a group of formula (III) 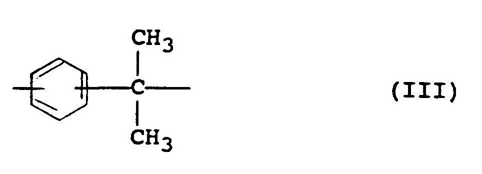 and Z is glycidoxy; the ratio of residues of formula (VIIIa) and (VIIIb) is selected so that the halogen-containing epoxy resin has an epoxy equivalent of 300 to 2000 and a halogen content of 5 to 30% by weight.

15. A process for producing an epoxy resin according to any one of claims 4 to 14 comprising reacting (A) a trifunctional epoxy compound of formula (I) and (B) a halogenated bisphenol in the presence of a catalyst.

16. A process according to claim 15 comprising condensing a bisphenol with an epihalohydrin and reacting the condensation product with (A) a trifunctional compound of formula (I) and (B) a halogenated bisphenol in the presence of a catalyst.

17. A resin composition comprising an epoxy resin as defined in any one of claims 4 to 14 or produced according to claim 15 to 16.

18. A process for producing a heat resistant article comprising Curing an epoxy resin as defined in any one of claims 4 to 14 or produced according to claim 15 or 16 or curing a resin composition according to claim 17.

## Patentansprüche

1. Trifunktionelle Epoxyverbindung der Formel (I) 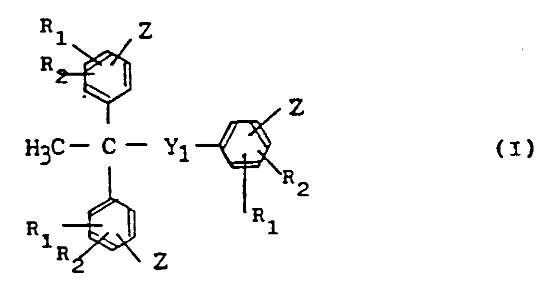 worin R₁ und R₂ gleich oder verschieden sind und jedes Wasserstoff, Halogen, C₁-C₄-Alkyl oder C₁-C₄-Alkoxy bedeutet, die Gruppen R₁ und R₂ die an die jeweiligen Phenylgruppen gebunden sind können gleich oder verschieden sein, Z ist eine Gruppe der Formel (II): 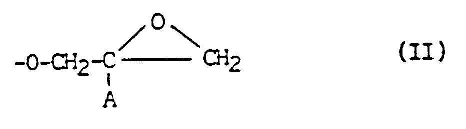 worin A Wasserstoff oder Methyl bedeutet, und Y₁ stellt eine Gruppe der Formel (III) 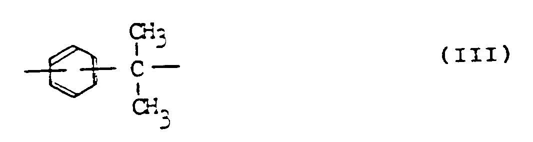 dar.

2. 1-[α-Methyl-α-(4'-glycidoxyphenyl)ethyl]-4-[α',α'-bis(4''-glycidoxyphenyl)ethyl]benzol.

3. 1-[α-Methyl-α-(3',5'-dimethyl-4'-glycidoxyphenyl)ethyl]-4-[α',α'-bis(3'',5''-dimethyl-4''-glycidoxyphenyl)ethyl]benzol.

4. Epoxyharz, umfassend Reste von
(A) einer trifunktionellen Epoxyverbindung der Formel (I): 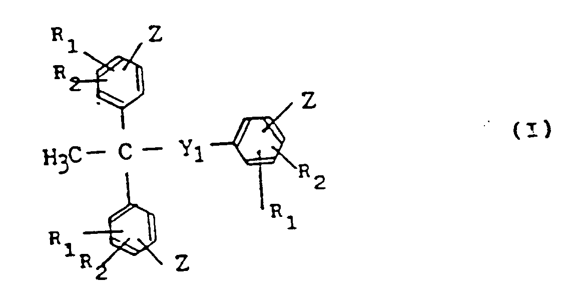 worin R₁ und R₂ gleich oder verschieden sind und jedes Wasserstoff, Halogen, C₁-C₄-Alkyl oder C₁-C₄-Alkoxy bedeutet, die Gruppen R₁ und R₂ die an die jeweiligen Phenylgruppen gebunden sind können gleich oder verschieden sein, Z ist eine Gruppe der Formel (II): 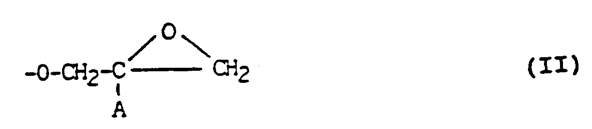 worin A Wasserstoff oder Methyl bedeutet und Y₁ stellt eine Gruppe der Formel (III) 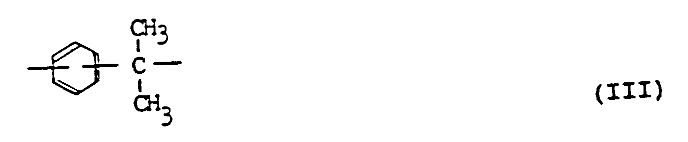 dar, und
(B) Reste eines halogenierten Bisphenols.

5. Epoxyharz gemäss Anspruch 4, umfassend Reste des difunktionellen Epoxykondensationsproduktes eines Bisphenols und ein Epihalohydrin.

6. Epoxyharz gemäss Anspruch 4 oder 5, worin die trifunktionelle Epoxyverbindung eine Verbindung der Formel (I) ist, worin Z Glycidoxy bedeutet, R₁ und R₂ gleich oder verschieden sind und jedes ist Wasserstoff oder C₁-C₄-Alkyl und Y₁ die im Anspruch 4 angegebene Bedeutung hat.

7. Epoxyharz gemäss einem der Ansprüche 4 bis 6, welches ein Epoxyäquivalent von 300 oder 2000 aufweist.

8. Epoxyharz gemäss einem der Ansprüche 4 bis 7, welches einen Halogengehalt von 5 bis 30 Gew.% aufweist.

9. Epoxyharz gemäss einem der Ansprüche 4 bis 8, worin das halogenierte Bisphenol Tetrabrombisphenol A ist.

10. Epoxyharz gemäss einem der Ansprüche 5 bis 9, worin das Gewichtsverhältnis der Reste der trifunktionellen Epoxyverbindung zu den Resten des difunktionellen Epoxy Kondensationsproduktes im Bereich von 50/50 bis 90/10 liegt.

11. Epoxyharz gemäss Anspruch 10, worin das Gewichtsverhältnis der Reste der trifunktionellen Epoxyverbindung zu den Resten des Kondensationsproduktes im Bereich von 60/40 bis 80/20 liegt.

12. Epoxyharz gemäss einem der Ansprüche 4 bis 11, welches in Methylethylketon löslich ist.

13. Epoxyharz der Formel (VI) 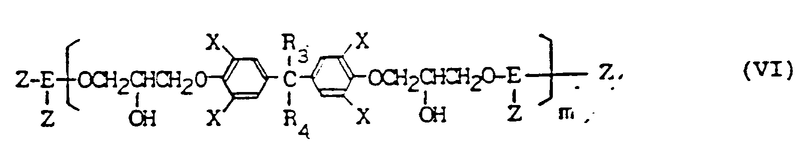 worin Z Glycidoxy bedeutet, R₃ und R₄ gleich oder verschieden sind und jedes Wasserstoff oder C₁-C₄-Alkyl bedeutet, X ist Halogen, E ist eine Gruppe der Formel (VIII) 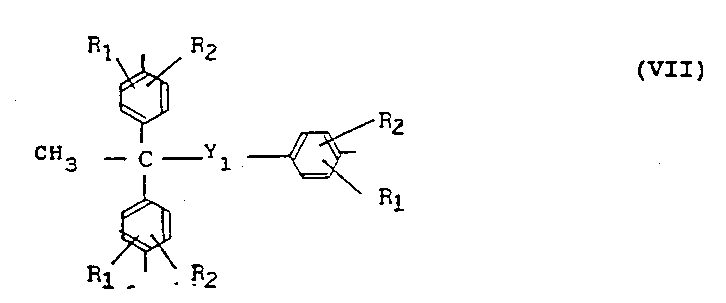 worin R₁ und R₂ gleich oder verschieden sind, und jedes bedeutet Wasserstoff oder C₁-C₄-Alkyl, die Gruppen R₁ und R₂ die an die jeweiligen Phenylgruppen gebunden sind können gleich oder verschieden sein, Y₁ ist eine Gruppe der Formel (III) 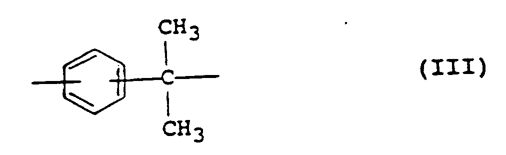 und m ist eine ganze Zahl von mindestens 1, welches ein Epoxyäquivalent von 300 bis 2000 und einen Halogengehalt von 5 bis 30 Gew.% aufweist.

14. Epoxyharz umfassend Reste der Formel (VIIIa): 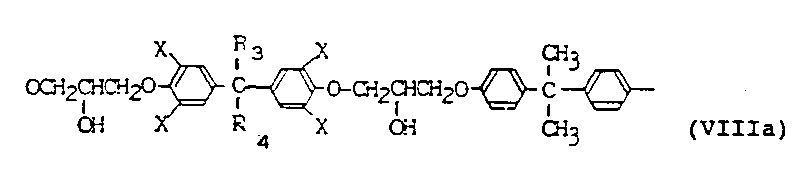 worin R₃ und R₄ gleich oder verschieden sind und jedes Wasserstoff oder C₁-C₄-Alkyl bedeutet, X ist Halogen, und Reste der Formel (VIIIb) 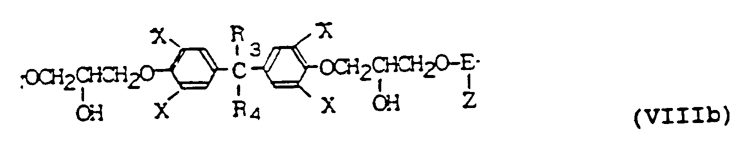 worin R₃ und R₄ die oben angegebene Bedeutung haben, E eine Gruppe der Formel (VII) 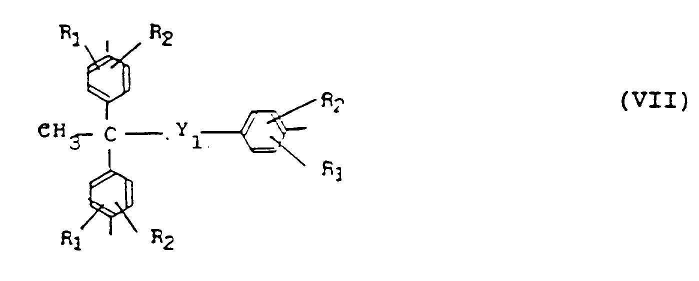 darstellt, worin R₁ und R₂ gleich oder verschieden sind und jedes Wasserstoff oder C₁-C₄-Alkyl bedeutet, die Gruppen R₁ und R₂ die an die jeweiligen Phenylgruppen gebunden sind können gleich oder verschieden sein, Y₁ ist eine Gruppe der Formel (III) 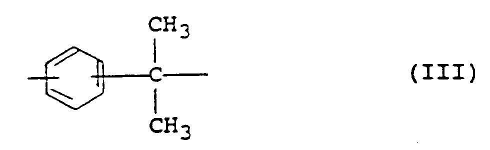 und Z ist Glycidoxy, wobei das Verhältnis der Reste der Formel (VIIIa) und (VIIIb) so ausgewählt ist, dass das Halogen enthaltende Epoxyharz ein Epoxyäquivalent von 300 bis 2000 und einen Halogengehalt von 5 bis 30 Gew.% aufweist.

15. Verfahren zur Herstellung eines Epoxyharzes gemäss einem der Ansprüche 4 bis 14, umfassend, die Reaktion von (A) einer trifunktionellen Epoxyverbindung der Formel (I) und (B) eines halogenierten Bisphenols in Anwesenheit eines Katalysators.

16. Verfahren gemäss Anspruch 15, umfassend kondensieren ein Bisphenol mit einem Epihalohydrin und das Kondensationsprodukt mit (A) einer trifunktionellen Verbindung der Formel (I) und (B) einem halogenierten Bisphenol in Anwesenheit eines Katalysators zur Reaktion bringen.

17. Harzzusammensetzung, umfassend, ein Epoxyharz wie in einem der Ansprüche 4 bis 14 definiert oder hergestellt gemäss Anspruch 15 bis 16.

18. Verfahren zur Herstellung eines wärmebeständigen Gegenstandes, umfassend, Härtung eines Epoxyharzes wie in einem der Ansprüche 4 bis 14 definiert, oder hergestellt gemäss Anspruch 15 oder 16 oder Härtung einer Harzzusammensetzung gemäss Anspruch 17.

## Revendications

1. Composé époxy trifonctionnel, de formule (I) : 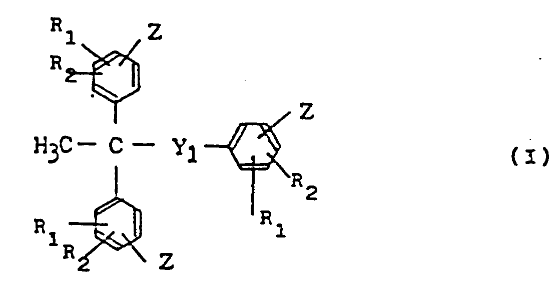 dans laquelle R₁ et R₂ sont identiques ou différents et représentent chacun un atome d'hydrogène ou d'halogène, ou un groupe alkyle ayant 1 à 4 atomes de carbone ou alcoxy ayant 1 à 4 atomes de carbone, les groupes R₁ et R₂ liés aux divers groupes phényle pouvant être identiques ou différents, Z représente un groupe de formule (II) : 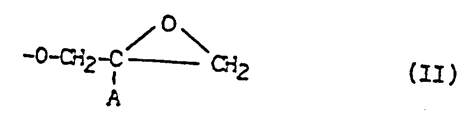 dans laquelle A représente un atome d'hydrogène ou un groupe méthyle, et Y₁ représente un groupe de formule (III) :

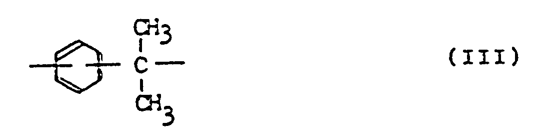

2. Le composé 1-[α-méthyl-α-(4'-glycidoxyphényl)éthyl]-4-[α',α'-bis(4''-glycidoxyphényl)éthyl]benzène.

3. Le composé 1-[α-méthyl-α-(3',5'-diméthyl-4'-glycidoxyphényl)éthyl]-4-[α',α'-bis(3'',5''-diméthyl-4''-glycidoxyphényl)éthyl]benzène.

4. Résine époxy comprenant des restes de (A) un composé époxy trifonctionnel de formule (I): 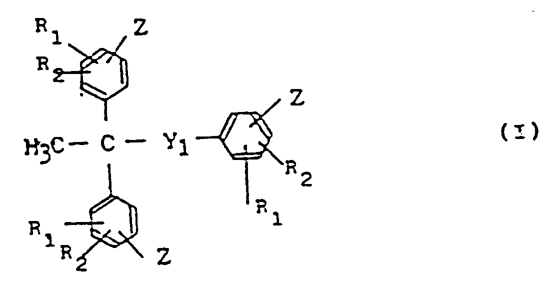 dans laquelle R₁ et R₂ sont identiques ou différents et représentent chacun un atome d'hydrogène ou d'halogène, ou un groupe alkyle ayant 1 à 4 atomes de carbone ou alcoxy ayant 1 à 4 atomes de carbone, les groupes R₁ et R₂ liés aux divers groupes phényle pouvant être identiques ou différents, Z représente un groupe de formule (II) : 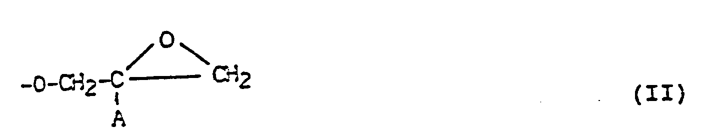 dans laquelle A représente un atome d'hydrogène ou un groupe méthyle, et Y₁ représente un groupe de formule (III) : 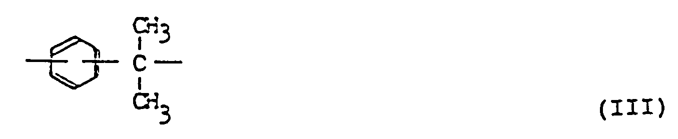 et des restes de (B) un bisphénol halogéné.

5. Résine époxy selon la revendication 4, comprenant des restes du produit de condensation époxy bifonctionnel d'un bisphénol et d'une épihalogénhydrine.

6. Résine époxy selon la revendication 4 ou 5, dans laquelle le composé époxy trifonctionnel est un composé de formule (I) dans laquelle Z représente un groupe glycidoxy, R₁ et R₂ sont identiques ou différents et représentent chacun un atome d'hydrogène ou un groupe alkyle ayant 1 à 4 atomes de carbone, et Y₁ est tel que défini dans la revendication 4.

7. Résine époxy selon l'une quelconque des revendications 4 à 6, ayant un équivalent époxyde de 300 à 2000.

8. Résine époxy selon l'une quelconque des revendications 4 à 7, ayant une teneur en halogène de 5 à 30% en poids.

9. Résine époxy selon l'une quelconque des revendications 4 à 8, dans laquelle le bisphénol halogéné est le tétrabromobisphénol A.

10. Résine époxy selon l'une quelconque des revendications 5 à 9, dans laquelle le rapport en poids des restes du composé époxy trifonctionnel aux restes du produit de condensation époxy bifonctionnel est compris dans l'intervalle allant de 50/50 à 90/10.

11. Résine époxy selon la revendication 10, dans laquelle le rapport en poids des restes du composé époxy trifonctionnel aux restes du produit de condensation est compris dans l'intervalle allant de 60/40 à 80/20.

12. Résine époxy selon l'une quelconque des revendications 4 à 11, qui est soluble dans la méthyléthylcétone.

13. Résine époxy de formule (VI) : 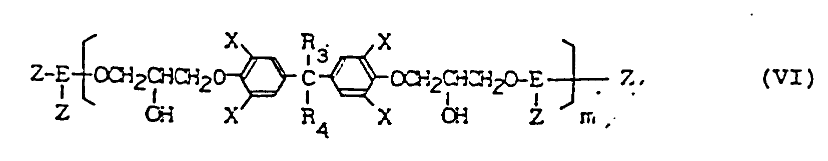 dans laquelle Z représente un groupe glycidoxy, R₃ et R₄ sont identiques ou différents et représentent chacun un atome d'hydrogène ou un groupe alkyle ayant 1 à 4 atomes de carbone, X représente un atome d'halogène, E représente un groupe de formule (VII) : 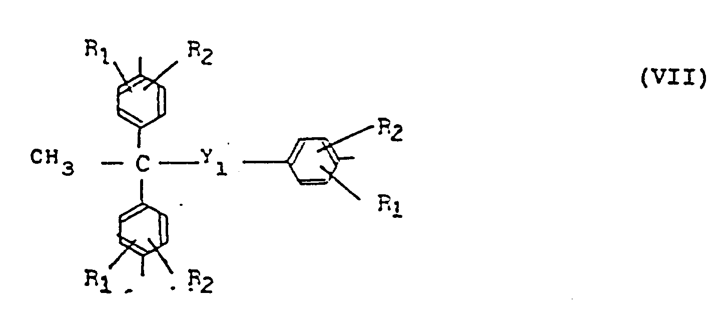 dans laquelle R₁ et R₂ sont identiques ou différents et représentent chacun un atome d'hydrogène ou un groupe alkyle ayant 1 à 4 atomes de carbone, les groupes R₁ et R₂ liés aux divers groupes phényle pouvant être identiques ou différents, et Y₁ est un groupe de formule (III) : 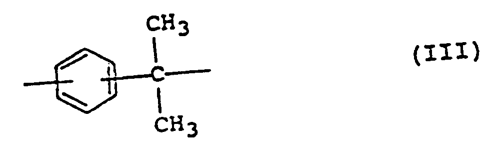 et m est un nombre entier égal à au moins 1, ladite résine ayant un équivalent époxyde de 300 à 2000 et une teneur en halogène de 5 à 30 % en poids.

14. Résine époxy comprenant des restes de formule (VIIIa) : 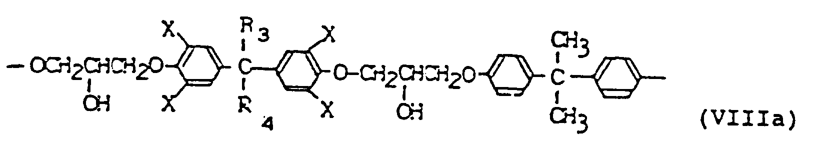 dans laquelle R₃ et R₄ sont identiques ou différents et représentent chacun un atome d'hydrogène ou un groupe alkyle ayant 1 à 4 atomes de carbone, et X représente un atome d'halogène, et des restes de formule (VIIIb) : 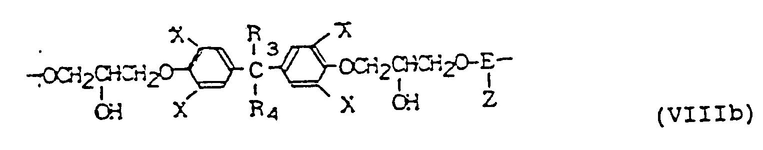 dans laquelle R₃ et R₄ sont tels que définis précédemment, E représente un groupe de formule (VII) : 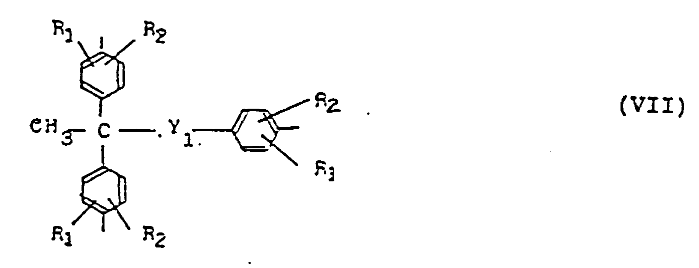 dans laquelle R₁ et R₂ sont identiques ou différents et représentent chacun un atome d'hydrogène ou un groupe alkyle ayant 1 à 4 atomes de carbone, les groupes R₁ et R₂ liés aux divers groupes phényle pouvant être identiques ou différents, et Y₁ représente un groupe de formule (III) : 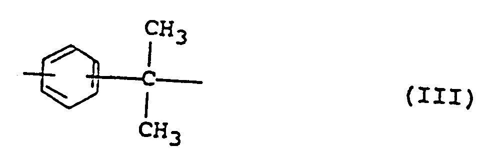 et Z représente un groupe glycidoxy,
le rapport des restes de formule (VIIIa) aux restes de formule (VIIIb) étant choisi de telle manière que la résine époxy halogénée ait un équivalent époxyde de 300 à 2000 et une teneur en halogène de 5 à 30 % en poids.

15. Procédé de préparation d'une résine époxy selon l'une quelconque des revendications 4 à 14, comprenant la réaction de (A) un composé époxy trifonctionnel de formule (I) et de (B) un bisphénol halogéné, en présence d'un catalyseur.

16. Procédé selon la revendication 15, comprenant la condensation d'un bisphénol avec une épihalogènhydrine et la réaction du produit de condensation avec (A) un composé trifonctionnel de formule (I) et (B) un bisphénol halogéné, en présence d'un catalyseur.

17. Composition de résine comprenant une résine époxy telle que définie dans l'une quelconque des revendications 4 à 14 ou produite par le procédé selon la revendication 15 ou 16.

18. Procédé de préparation d'un article résistant à la chaleur, comprenant le durcissement d'une résine époxy telle que définie dans l'une quelconque des revendications 4 à 14 ou produite par le procédé selon la revendication 15 ou 16, ou le durcissement d'une composition de résine selon la revendication 17.
